# EUROPÄISCHE PATENTSCHRIFT

(11) **EP 1 586 273 B1**
(45) Veröffentlichungstag und Bekanntmachung des Hinweises auf die Patenterteilung: **15.04.2009**
(21) Anmeldenummer: 05008299.9
(22) Anmeldetag: 15.04.2005
(51) Int. Cl.: A61B 17/16, H03K 17/97

(54) **Chirurgische Drückereinheit und chirurgische Antriebseinheit**
Surgical control button and drive units
Unités de boutons de commande et d'entraînement chirurgicales

(30) Priorität: 16.04.2004 DE 102004020808
(43) Veröffentlichungstag der Anmeldung: 19.10.2005
(73) Patentinhaber: Aesculap AG, 78532 Tuttlingen (DE)
(72) Erfinder: Hoegerle, Roland Alois, 78532 Tuttlingen (DE)
(74) Vertreter: Hoeger, Stellrecht & Partner Patentanwälte

(56) Entgegenhaltungen:
- EP-A- 1 236 439
- DE-U-9202004 006 65
- DE-U-4202004 006 72
- GB-A- 1 364 437
- US-A- 5 747 953
- US-A1- 2004 022 527

## Beschreibung

Die vorliegende Erfindung betrifft eine chirurgische Drückereinheit zum Vorgeben einer Drehzahl und/oder einer Drehrichtung einer chirurgischen Antriebseinheit, umfassend mindestens ein in einer Betätigungsrichtung beweglich gelagertes Betätigungsglied, eine Felderzeugungseinheit zum Erzeugen eines magnetischen oder elektrischen Feldes und mindestens einen Betätigungssensor zum Erzeugen eines Betätigungssignals als Reaktion auf eine Bewegung und/eine Stellung des Betätigungsglieds, wobei das erzeugte Betätigungssignal mit einer Feldstärke und/oder einer Änderung des von der Felderzeugungseinheit erzeugten Feldes in Folge einer Bewegung des Betätigungsgliedes korreliert ist.

Ferner betrifft die vorliegende Erfindung eine chirurgische Antriebseinheit umfassend einen chirurgischen Antrieb und eine Drückereinheit zum Vorgeben einer Drehzahl und/oder einer Drehrichtung der Antriebseinheit.

Aus der US 5,747,953 ist beispielsweise eine batteriebetriebene chirurgische Antriebseinheit mit einer Drückereinheit in Form einer Akkumaschine bekannt, welche als Felderzeugungseinheit einen Magneten aufweist, welcher mit einem Drücker der Drückereinheit verbunden ist und relativ zu einem als Betätigungssensor dienenden Hallsensor bewegt werden kann. Durch Verändern der räumlichen Distanz des Magneten zum Sensor wird ein Betätigungssignal erzeugt. Eine derartige Anordnung ermöglicht es zwar, zu Reinigungszwecken, beispielsweise zum Sterilisieren der Antriebseinheit, eine in der Antriebseinheit angeordnete Elektronik vor dem Sterilisieren von der Drückereinheit zu trennen. Allerdings weist die bekannte Konstruktion den Nachteil auf, daß der Magnet einen Reinigungszyklus ebenfalls mitmachen muß. Insbesondere dann, wenn er nicht vollständig gekapselt ist, kann er sich unter Umständen von dem Drücker lösen. Ferner stellt sich bei jeder Verbindung der Elektronik, welche den Hallsensor umfaßt, und den Drückern der Drückereinheit das Problem, daß Magnet und Sensor relativ zueinander wieder richtig justiert werden. Wird die Antriebseinheit häufig zerlegt, dann kann es im Laufe der Zeit zu einer Dejustage kommen, die die Funktionsfähigkeit der Antriebseinheit negativ beeinflussen kann.

Aus der GB-A-1364437 ist eine magnetische Schaltvorrichtung bekannt, welche einen Permanentmagneten umfasst, der so ausgebildet ist, dass er einen magnetischen Schaltkörper umschließt und dass eine Magnetisierung des magnetischen Schaltkörpers durch Bewegen eines Feldänderungsglieds in den Permanentmagnet hinein beziehungsweise aus diesem heraus geändert werden kann.

Dementsprechend ist es Aufgabe der vorliegenden Erfindung eine chirurgische Drückereinheit und eine chirurgische Antriebseinheit der eingangs beschriebenen Art so zu verbessern, daß die Betriebssicherheit der Antriebseinheit langfristig sichergestellt wird.

Diese Aufgabe wird bei einer chirurgischen Drückereinheit erfindungsgemäß dadurch gelöst, daß der Betätigungssensor mit der Felderzeugungseinheit gekoppelt ist, daß ein Feldänderungsglied vorgesehen ist zum Erzeugen einer Änderung des am Ort des Betätigungssensors wirkenden, von der Felderzeugungseinheit erzeugten Feldes infolge einer Bewegung und/oder einer geänderten Stellung des Betätigungsgliedes und daß die Felderzeugungseinheit über ein Rückschlußsystem mit dem Betätigungssensor gekoppelt ist.

Diese Ausgestaltung macht es insbesondere möglich, den Betätigungssensor zusammen mit der Felderzeugungseinheit in einer festen räumlichen Korrelation zu Reinigungszwecken aus der Antriebseinheit zu entnehmen, so daß keine Toleranzprobleme beim Zusammensetzen von Antriebseinheit und Steuerelektronik mit Felderzeugungseinheit und Betätigungssensor auftreten können. Insbesondere dann, wenn bei mehreren verwendeten Antriebseinheiten deren Steuerelektroniken vertauscht werden, wirkt sich dies nicht nachteilig aus. Zudem läßt sich so vermeiden, daß die Felderzeugungseinheit einer Aufbereitung der Antriebseinheit unterzogen wird. Ferner wird die Betriebssicherheit erhöht, da die Felderzeugungseinheit nicht mehr zwingend relativ zum Sensor bewegt werden muß. Eine besonders gute Kopplung zwischen der Felderzeugungseinheit und dem Betätigungssensor kann dadurch erreicht werden, daß die Felderzeugungseinheit über ein Rückschlußsystem mit dem Betätigungssensor gekoppelt ist. Das Rückschlußsystem eignet sich in vorteilhafter Weise dazu, einen Fluß des von der Felderzeugungseinheit erzeugten Feldes direkt von der Felderzeugungseinheit zum Betätigungssensor zu führen. Dadurch werden unerwünschte Einflüsse des von der Felderzeugungseinheit erzeugten Feldes auf eine Steuerelektronik der Antriebseinheit vermieden.

Vorzugsweise sind der Betätigungssensor und die Felderzeugungseinheit relativ zueinander feststehend angeordnet. So lassen sich alle möglichen Nachteile einer relativ zueinander beweglichen Anordnung von Betätigungssensor und Felderzeugungseinheit gänzlich ausschließen. Eine Funktionsfähigkeit der Drückereinheit bleibt gewährleistet, da das Feldänderungsglied eine Änderung des von der Felderzeugungseinheit erzeugten Feldes am Ort des Betätigungssensors bewirken kann, zur Verwendung mit der Antriebseinheit.

Grundsätzlich könnte der Betätigungssensor ein elektrischer oder ein elektromagnetischer Betätigungssensor sein. Vorzugsweise ist er jedoch ein Magnetfeldsensor. Damit eignet sich jede Felderzeugungseinheit, welche ein magnetisches Feld erzeugen kann, zur Verwendung mit der Antriebseinheit.

Vorzugsweise ist der Magnetfeldsensor ein Hallsensor. Mit einem solchen Sensor lassen sich auf einfache Weise Betätigungssignale in Form von elektrischen Spannungen erzeugen.

Vorteilhaft ist es, wenn das Feldänderungsglied mindestens teilweise magnetisch polarisierbar und eine von Null verschiedene magnetische Suszeptibilität χₘ aufweist. Dies ermöglicht es, bei Einführen des Feldänderungsglieds in das von der Felderzeugungseinheit erzeugte Feld, daß eine Flußdichte am Ort des Betätigungssensors verändert wird, wodurch ein Betätigungssignal erzeugt werden kann.

Besonders günstig ist es, wenn das Feldänderungsglied mindestens.teilweise diamagnetisch, paramagnetisch, ferromagnetisch, antiferromagnetisch oder ferrimagnetisch ist. Materialien mit den genannten magnetischen Eigenschaften können eine gewünschte Änderung eines Magnetfeldes, insbesondere am Ort eines Magnetfeldsensors, bewirken.

Ein besonders einfacher Aufbau der Drückereinheit ergibt sich, wenn das Feldänderungsglied ein Weicheisenelement ist. Es läßt sich auf einfache Weise herstellen und ist widerstandsfähig gegen herkömmliche Reinigungsmittel, welche zur Reinigung der Antriebseinheit eingesetzt werden.

Besonders einfach wird der Aufbau der Drückereinheit, wenn die Felderzeugungseinheit ein Magnet ist.

Grundsätzlich wäre es denkbar, als Magnet einen Elektromagnet einzusetzen. Besonders sicher wird ein Betrieb der Drückereinheit jedoch dann, wenn der Magnet ein Permanentmagnet ist. Wartungsintervalle der Drückereinheit werden dadurch deutlich verlängert.

Vorteilhaft ist es aber auch, wenn der Magnet durch eine Spule gebildet wird.

Um eine möglichst hohe Flußdichte am Ort des Betätigungssensors zu erhalten, ist es vorteilhaft, wenn der Betätigungssensor zwischen Polen der Felderzeugungseinheit angeordnet ist.

Insbesondere kann es gemäß einer bevorzugten Ausführungsform der Erfindung vorteilhaft sein, wenn der Betätigungssensor in einem Spalt einer Ringspule angeordnet ist.

Um zusätzlich eine Verstärkung des Betätigungssignals zu erhalten, ist es günstig, wenn ein Querschnitt des Feldänderungsglieds in einer Betätigungsrichtung des Betätigungsglieds variiert. Dadurch wird erreicht, daß das von einem Fluß des Feldes durchsetzte Volumen des Feldänderungsglieds in Folge einer Betätigung des mindestens einen Betätigungsglieds vergrößert wird, nämlich dann, wenn das Feldänderungsglied in das Feld der Felderzeugungseinheit hinein- oder aus dem Feld herausbewegt wird.

Günstigerweise nimmt der Querschnitt zu. Ein Feldänderungsglied mit einem zunehmenden Querschnitt läßt sich besonders leicht ausbilden.

Insbesondere bei einer magnetischen Felderzeugungseinheit ist es günstig, wenn das Rückschlußsystem ein magnetisches Rückschlußsystem ist. Beispielsweise kann hier ein magnetisierbares Material zur Herstellung des Rückschlußsystems, beispielsweise einer mechanischen Kopplung, verwendet werden.

Gemäß einer bevorzugten Ausführungsform der Erfindung kann ferner vorgesehen sein, daß die Felderzeugungseinheit, das Rückschlußsystem und der Betätigungssensor eine Ausnehmung definieren und daß das Feldänderungsglied derart angeordnet ist, daß es in Folge einer Bewegung des Betätigungsglieds in die Ausnehmung mindestens teilweise einführbar ist. So läßt sich einfach und sicher eine Änderung des am Ort des Betätigungssensors wirkenden Feldes bewirken.

Der Aufbau der Drückereinheit wird besonders einfach, wenn die Ausnehmung einen im wesentlichen rechteckigen Querschnitt aufweist.

Grundsätzlich wäre es denkbar, daß das Betätigungsglied das Feldänderungsglied antreibt oder mit diesem indirekt verbunden ist. Der Aufbau der Drückereinheit wird jedoch noch einfacher, wenn das Betätigungsglied das Feldänderungsglied trägt.

Vorteilhaft ist es, wenn die Antriebseinheit mindestens zwei verschiedene Betriebsmodi aufweist, wobei einem ersten Betriebsmodus eine erste Betriebsdmodistellung des mindestens einen Betätigungsglieds zugeordnet ist und wenn das mindestens eine Betätigungsglied von der ersten Betriebsmodistellung in eine zweite Betriebsmodistellung, welche einem zweiten Betriebsmodus der Antriebseinheit zugeordnet ist, um eine Drehachse verdrehbar ist zum Umschalten der Antriebseinheit vom ersten Betriebsmodus in den zweiten Betriebsmodus. Eine solche Ausgestaltung der Drückereinheit gestattet es, dem Betätigungsglied mehrere Funktionen zuzuweisen. Beispielsweise kann es als Drehzahlvorgabeglied zum Vorgeben einer Drehzahl der Antriebseinheit dienen und gleichzeitig als Umschalter von einem ersten in einen zweiten Betriebsmodus.

Günstig kann es sein, wenn zwei Betätigungssensoren vorgesehen und derart angeordnet sind, daß mit dem einen Betätigungssensor eine Stellung des mindestens einen Betätigungsglieds in der ersten Betriebsmodistellung und/oder eine Bewegung des mindestens einen Betätigungsglieds in ersten Betriebsmodistellung und daß mit dem zweiten Betätigungssensor eine Stellung des mindestens einen Betätigungsglieds in der zweiten Betriebsmodistellung und/oder eine Bewegung des mindestens einen Betätigungsglieds in der zweiten Betriebsmodistellung detektierbar ist. Man kann auf diese Weise in Abhängigkeit von einer erforderlichen Empfindlichkeit unterschiedliche Betätigungssensoren für unterschiedliche Zwecke einsetzen. So läßt sich der Aufbau der Drückereinheit an besondere Erfordernisse einer verwendeten Schaltung oder deren Bauelemente anpassen.

Die eingangs gestellte Aufbau wird ferner bei einer chirurgischen Antriebseinheit der eingangs beschriebenen Art erfindungsgemäß dadurch gelöst, daß die Drückereinheit eine der oben beschriebenen Drückereinheiten ist. Die oben im Zusammenhang mit unterschiedlichen Ausführungsformen der Drückereinheit beschriebenen Vorteile sind somit direkt auch auf die Antriebseinheit übertragbar und machen einen Betrieb derselben besonders einfach und sicher.

Grundsätzlich könnte die Antriebseinheit eine schnurgebundene, mit einem Steuergerät verbundene Antriebseinheit sein. Um einen universellen Einsatz der Antriebseinheit in einem Operationssaal zu gestatten, ist es vorteilhaft, wenn die Antriebseinheit batterie- oder akkubetrieben ist.

Vorteilhaft ist es, wenn eine Steuer- und/oder Regelungseinheit vorgesehen ist zum Steuern und/oder Regeln einer Drehzahl und/oder eines Betriebsmodus der Antriebseinheit. Ein Betätigungssignal des Betätigungssensors läßt sich so in gewünschter Weise aufbereiten, um die Antriebseinheit mit einer von einem Bediener vorgegebenen Drehzahl und einem Betriebsmodus zu betreiben.

Eine besonders kompakte Antriebseinheit läßt sich ausbilden, wenn diese die Steuer- und/oder Regelungseinheit umfaßt.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische, teilweise geschnittene Ansicht einer Antriebseinheit;
- Figur 2:: eine schematische Darstellung einer Drücker-/Sensoranordnung; und
- Figur 3:: eine schematische Darstellung eines relativ zum Betätigungssensor bewegten Feldänderungsglieds.

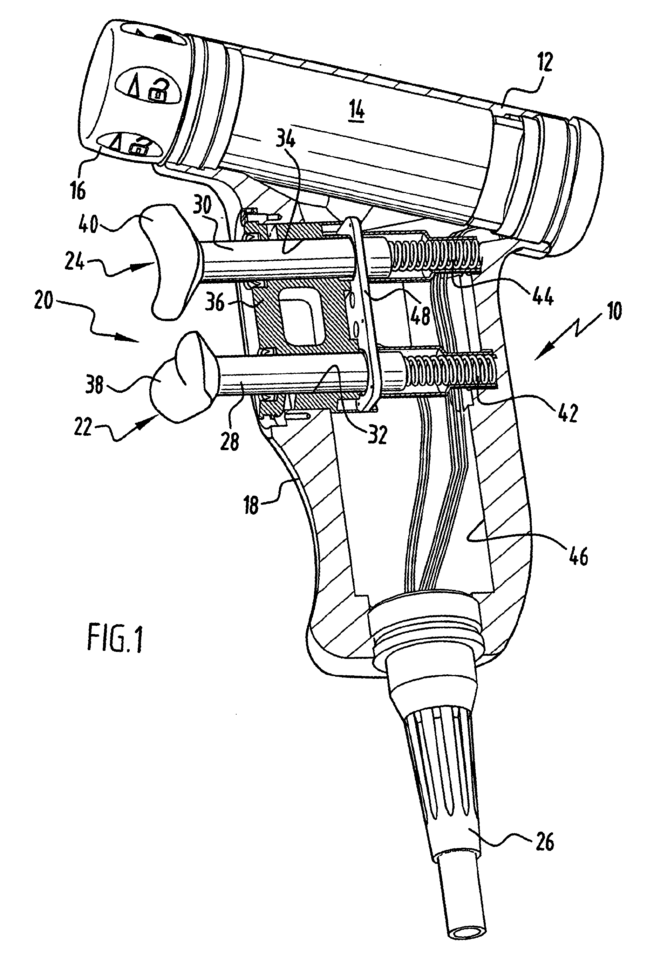

In Figur 1 ist eine insgesamt mit dem Bezugszeichen 10 versehene chirurgische Antriebsmaschine dargestellt, die einen in einem Gehäuse 12 angeordneten Motor 14 umfaßt und welche über eine Kupplung 16 mit einem nicht dargestellten Behandlungswerkzeug in Form eines chirurgischen Handstücks verbindbar ist.

An einen Abschnitt des Gehäuses 12, welcher den Motor 14 umgibt, schließt sich ein Griffbereich 18 an, an welchem eine insgesamt mit dem Bezugszeichen 20 versehene Drückereinheit angeordnet ist, die zwei Drücker 22 und 24 aufweist. Über ein Anschlußkabel 26 ist die Antriebsmaschine 10 mit einem nicht dargestellten Steuergerät verbindbar, mit welchem von der Drückereinheit 20 erzeugte Betätigungssignale zum Ansteuern und/oder Regeln des Motors 12 verarbeitet werden können.

Die beiden Drücker 22 und 24 der Drückereinheit 20 sind im wesentlichen identisch ausgebildet. Sie weisen beide einen zylindrischen Grundkörper 28 beziehungsweise 30 auf, welcher jeweils in einer Aufnahmebohrung 32 beziehungsweise 34 eines Rahmens 36 geführt ist. An aus dem Rahmen 36 abstehenden Enden sind auf die Grundkörper 28 und 30 jeweils Betätigungsknöpfe 38 und 40 aufgesetzt, welche ergonomisch ausgebildet sind. Die beiden Drücker 22 und 24 sind jeweils in der Aufnahmebohrung 32 beziehungsweise 34 längsverschieblich geführt und können in ausgewählten Stellungen um 90° oder 180° verdreht werden. Schraubenfedern 42 und 44, die sich einerseits an einer Innenwand 46 des Griffsbereichs 18 und andererseits an den Betätigungsknöpfen 38 und 40 abgewandten Enden der Grundkörper 28 und 30 abstützen, dienen als Kraftspeicher, gegen die die beiden Drücker 22 und 24 betätigt werden können.

An den Rahmen 36 schließt sich eine Leiterplatte 48 an, auf welcher jeweils ein nicht dargestellter Betätigungssensor für jeden der beiden Drücker 22 und 24 angeordnet ist.

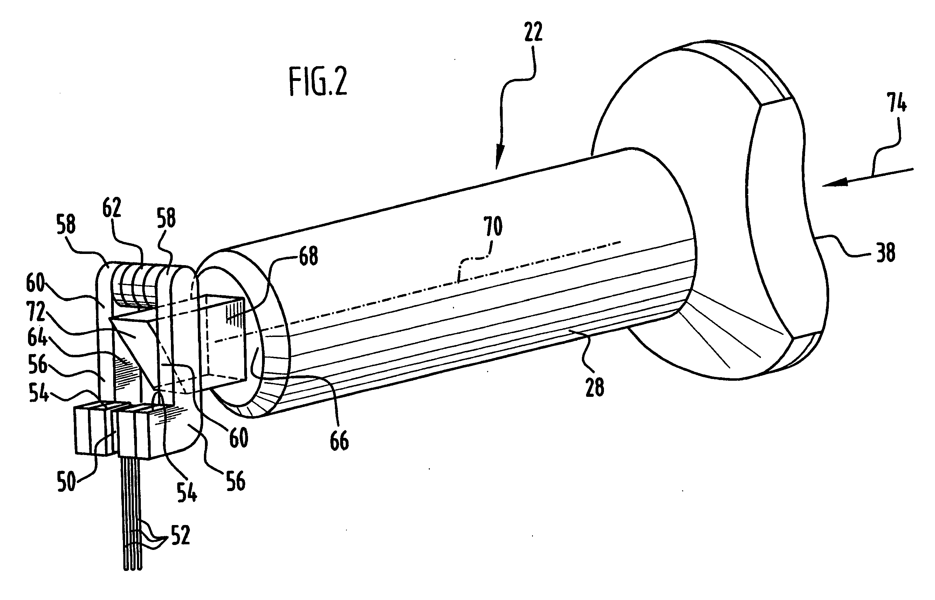

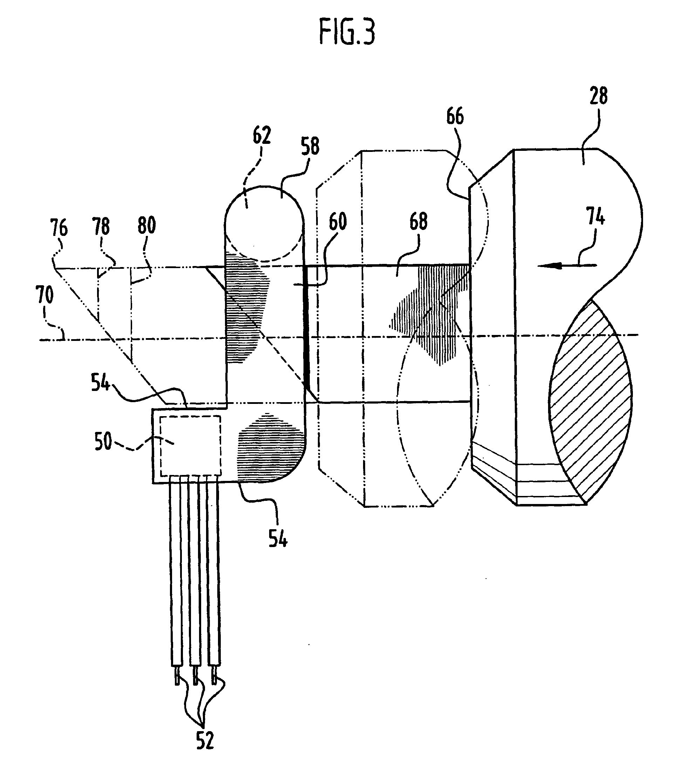

In den Figuren 2 und 3 ist schematisch dargestellt, wie mit dem Betätigungssensor ein Betätigungssignal erzeugt werden kann.

Ein Hallsensor 50 mit drei Anschlußkontakten 52 dient als Betätigungssensor. Er ist zwischen kurzen Schenkeln 54 zweier L-förmiger Weicheisenplatten 56 eingebracht, so daß sich eine quaderförmige Struktur aus den Schenkeln 54 und dem dazwischen angeordneten Hallsensor, 50 und gegebenenfalls weiterer, einen Zwischenraum zwischen den Schenkeln 54 ausfüllenden Weicheisenelementen ergibt. Zwischen freien Enden 58 zweier langer Schenkel 60 der beiden Weicheisenplatten 56 ist ein zylindrischer Stabmagnet 62 angeordnet. Insgesamt ergibt sich also eine rahmenförmige Struktur, die eine von den langen Schenkeln 60, dem Stabmagnet 62 und den kurzen Schenkeln 54 mit dem dazwischenliegenden Hallsensor 50 eine Öffnung 64 definiert, welche einen rechteckigen Querschnitt aufweist. An dem in Figur 2 schematisch dargestellten Drücker 22 ist an einer vom Betätigungsknopf 38 weg weisenden Stirnfläche 66 des Grundkörpers 28 ein Weicheisenquader in Richtung einer Längsachse 70 des Grundkörpers 28 abstehend angeordnet, wobei eine Stirnfläche 72 des Weicheisenquaders 68 relativ zur Stirnfläche 66 des Grundkörpers 28 um 45° geneigt ist.

Die Rahmenstruktur mit dem Hallsensor 50 ist relativ zum Drücker 22 so angeordnet, daß infolge einer Betätigung des Drückers 22 in Richtung des Pfeils 74 in Figur 2, also parallel zur Längsachse 70, der Grundkörper 28 mit dem Weicheisenquader 68 in die Öffnung 64 hineinbewegt werden kann. Der ferromagnetische Weicheisenquader 68 weist aufgrund seiner Form ausgehend von einer vorderen Stirnkante 76 parallel zur Stirnfläche 66 zunehmende Querschnitte auf, was in Figur 3 durch die beiden eingezeichneten Schnittebenen 78 und 80 angedeutet ist, welche beide parallel zur Stirnfläche 66 verlaufen.

Wird der Weicheisenquader 68 in die Öffnung 64 hineinbewegt, dann hat dies Auswirkungen auf den magnetischen Fluß innerhalb des durch die beiden Weicheisenplatten 56 gebildeten Rückschlußsystems, welches den Stabmagnet 62 mit dem Hallsensor 50 koppelt. Eine Änderung des den Hallsensor 50 durchsetzenden magnetischen Flusses führt zu einer Änderung einer vom Hallsensor 50 erzeugten Hallspannung, welche über die Anschlußkontakte 52 abgegriffen werden kann. Vom Steuergerät wird dann die gemessene Hallspannung, welche als Betätigungssignal genutzt wird, in entsprechende Steuersignale für den Motor 14 aufbereitet.

Die beiden Drücker 22 und 24 sind derart angeordnet, daß sie insbesondere bei einer batteriebetriebenen Antriebsmaschine nicht zusammen mit einer Steuerelektronik und der Batterie vor einer Sterilisierung der Antriebsmaschine 10 entnommen werden können. An der Antriebsmaschine 10 verbleibt dann die Drückereinheit 20, welche die Feldänderungsglieder in Form der Weicheisenquader 68 umfaßt. Damit verbleiben keine elektrischen Kontakte innerhalb der Antriebsmaschine 10, die für die Erzeugung eines Betätigungssignals erforderlich sind, denn mit einer Steuerelektronik werden auch die Hallsensoren 50 vollständig entfernt. Die besondere Anordnung des Hallsensors 50 relativ zum Stabmagnet 62 gestattet es, deren räumliche Zuordnung eindeutig und dauerhaft festzulegen. Eine Nachjustierung wie bei herkömmlichen Systemen ist nicht erforderlich. Etwaige Fertigungstoleranzen bei der Größe und Form des Weicheisenquaders 68 und der Öffnung 64 wirken sich weit weniger kritisch auf eine Funktion der Drückereinheit 20 aus, als dies bei herkömmlichen Systemen mit einen relativ zu dem Hallsensor 50 bewegten Magneten der Fall ist.

## Patentansprüche

1. Chirurgische Drückereinheit (20) zum Vorgeben einer Drehzahl und/oder einer Drehrichtung einer chirurgischen Antriebseinheit (10), umfassend mindestens ein in einer Betätigungsrichtung (70) beweglich gelagertes Betätigungsglied (22, 24), eine Felderzeugungseinheit (62) zum Erzeugen eines magnetischen oder elektrischen Feldes und mindestens einen Betätigungssensor (50) zum Erzeugen eines Betätigungssignals als Reaktion auf eine Bewegung und/oder eine Stellung des Betätigungsglieds (22, 24), wobei das erzeugte Betätigungssignal mit einer Feldstärke und/oder einer Änderung des von der Felderzeugungseinheit (62) erzeugten Feldes infolge einer Bewegung des Betätigungsgliedes (22, 24) korreliert ist, **dadurch gekennzeichnet, daß** der Betätigungssensor (50) mit der Felderzeugungseinheit (62) gekoppelt ist, daß ein Feldänderungsglied (68) vorgesehen ist zum Erzeugen einer Änderung des am Ort des Betätigungssensors (50) wirkenden, von der Felderzeugungseinheit (62) erzeugten Feldes infolge einer Bewegung und/oder einer geänderten Stellung des Betätigungsgliedes (22, 24) und daß die Felderzeugungseinheit (62) über ein Rückschlußsystem (56) mit dem Betätigungssensor (50) gekoppelt ist.

2. Drückereinheit nach Anspruch 1, **dadurch gekennzeichnet, daß** der Betätigungssensor (50) und die Felderzeugungseinheit (62) relativ zueinander feststehend angeordnet sind.

3. Drückereinheit nach einem der voranstehenden Ansprüche, **dadurch gekennzeichnet, daß** der Betätigungssensor ein Magnetfeldsensor (50) ist.

4. Drückereinheit nach Anspruch 3, **dadurch gekennzeichnet, daß** der Magnetfeldsensor ein Hallsensor (50) ist.

5. Drückereinheit nach einem der voranstehenden Ansprüche, **dadurch gekennzeichnet, daß** das Feldänderungsglied (68) mindestens teilweise magnetisch polarisierbar ist und eine von Null verschiedene magnetische Suszeptibilität χₘ aufweist.

6. Drückereinheit nach Anspruch 5, **dadurch gekennzeichnet, daß** das Feldänderungsglied (68) mindestens teilweise diamagnetisch, paramagnetisch, ferromagnetisch, antiferromagnetisch oder ferrimagnetisch ist.

7. Drückereinheit nach Anspruch 5 oder 6, **dadurch gekennzeichnet, daß** das Feldänderungsglied ein Weicheisenelement (68) ist.

8. Drückereinheit nach einem der voranstehenden Ansprüche, **dadurch gekennzeichnet, daß** die Felderzeugungseinheit ein Magnet (62) ist.

9. Drückereinheit nach Anspruch 8, **dadurch gekennzeichnet, daß** der Magnet ein Permanentmagnet (62) ist.

10. Drückereinheit nach Anspruch 8, **dadurch gekennzeichnet, daß** der Magnet (62) durch eine Spule gebildet ist.

11. Drückereinheit nach einem der voranstehenden Ansprüche, **dadurch gekennzeichnet, daß** der Betätigungssensor (50) zwischen Polen der Felderzeugungseinheit (62) angeordnet ist.

12. Drückereinheit nach Anspruch 11, **dadurch gekennzeichnet, daß** der Betätigungssensor (50) in einem Spalt einer Ringspule angeordnet ist.

13. Drückereinheit nach einem der voranstehenden Ansprüche, **dadurch gekennzeichnet, daß** ein Querschnitt (78, 80) des Feldänderungsglieds (68) in einer Betätigungsrichtung (70) des Betätigungsglieds (22, 24) variiert.

14. Drückereinheit nach Anspruch 13, **dadurch gekennzeichnet, daß** der Querschnitt (78, 80) zunimmt.

15. Drückereinheit nach einem der voranstehenden Ansprüche, **dadurch gekennzeichnet, daß** das Rückschlußsystem (56) ein magnetisches Rückschlußsystem ist.

16. Drückereinheit nach einem der voranstehenden Ansprüche, **dadurch gekennzeichnet, daß** die Felderzeugungseinheit (62), das Rückschlußsystem (56) und der Betätigungssensor (50) eine Ausnehmung (64) definieren und daß das Feldänderungsglied (68) derart angeordnet ist, daß es infolge einer Bewegung des Betätigungsglieds (22, 24) in die Ausnehmung (64) mindestens teilweise einführbar ist.

17. Drückereinheit nach Anspruch 16, **dadurch gekennzeichnet, daß** die Ausnehmung (64) einen im wesentlich rechteckigen Querschnitt aufweist.

18. Drückereinheit nach einem der voranstehenden Ansprüche, **dadurch gekennzeichnet, daß** das Betätigungsglied (22, 24) das Feldänderungsglied (68) trägt.

19. Drückereinheit nach einem der voranstehenden Ansprüche, **dadurch gekennzeichnet, daß** die Antriebseinheit (10) mindestens zwei verschiedene Betriebsmodi aufweist, wobei einem ersten Betriebsmodus eine erste Betriebsmodistellung des mindestens einen Betätigungsglieds zugeordnet ist und daß das mindestens eine Betätigungsglied (22, 24) von der ersten Betriebsmodistellung in eine zweite Betriebsmodistellung, welche einem zweiten Betriebsmodus der Antriebseinheit (10) zugeordnet ist, um eine Drehachse (70) verdrehbar ist zum Umschalten der Antriebseinheit (10) vom ersten Betriebsmodus in den zweiten Betriebsmodus.

20. Drückereinheit nach Anspruch 19, **dadurch gekennzeichnet, daß** zwei Betätigungssensoren (50) vorgesehen und derart angeordnet sind, daß mit dem einen Betätigungssensor (50) eine Stellung des mindestens einen Betätigungsglieds (22, 24) in der ersten Betriebsmodistellung oder eine Bewegung des mindestens einen Betätigungsglieds (22, 24) in der ersten Betriebsmodistellung und daß mit dem zweiten Betätigungssensor eine Stellung des mindestens einen Betätigungsglieds (22, 24) in der zweiten Betriebsmodistellung oder eine Bewegung des mindestens einen Betätigungsglieds (22, 24) in der zweiten Betriebsmodistellung detektierbar ist.

21. Chirurgische Antriebseinheit (10) umfassend einen chirurgischen Antrieb (14) und eine Drückereinheit (20) zum Vorgeben einer Drehzahl und/oder einer Drehrichtung der Antriebseinheit, **dadurch gekennzeichnet, daß** die Drückereinheit (20) eine Drückereinheit (20) nach einem der voranstehenden Ansprüche ist.

22. Antriebseinheit nach Anspruch 21, **dadurch gekennzeichnet, daß** die Antriebseinheit (10) batterie- oder akkubetrieben ist.

23. Antriebseinheit nach einem der Ansprüche 21 oder 22, **dadurch gekennzeichnet, daß** eine Steuer- und/oder Regelungseinheit vorgesehen ist zum Steuern und/oder Regeln einer Drehzahl und/oder eines Betriebsmodus der Antriebseinheit (10).

24. Antriebseinheit nach Anspruch 23, **dadurch gekennzeichnet, daß** die Antriebseinheit (10) die Steuer- und/oder Regelungseinheit umfaßt.

## Claims

1. Surgical push-button unit (20) for prescribing a rotational speed and/or a direction of rotation of a surgical drive unit (10), comprising at least one actuating member (22, 24) mounted for movement in a direction of actuation (70), a field generating unit (62) for generating a magnetic or electric field, and at least one actuating sensor (50) for generating an actuation signal in response to a movement and/or a position of the actuating member (22, 24), the generated actuation signal being correlated with a field strength and/or a change in the field generated by the field generating unit (62), which occurs as a result of a movement of the actuating member (22, 24), **characterized in that** the actuating sensor (50) is coupled with the field generating unit (62), **in that** a field changing member (68) is provided for generating a change in the field acting at the location of the actuating sensor (50) and generated by the field generating unit (62), which occurs as a result of a movement and/or a changed position of the actuating member (22, 24), and **in that** the field generating unit (62) is coupled by a return path system (56) with the actuating sensor (50).

2. Push-button unit in accordance with claim 1, **characterized in that** the actuating sensor (50) and the field generating unit (62) are fixedly disposed relative to each other.

3. Push-button unit in accordance with any one of the preceding claims, **characterized in that** the actuating sensor is a magnetic field sensor (50).

4. Push-button unit in accordance with claim 3, **characterized in that** the magnetic field sensor is a Hall sensor (50).

5. Push-button unit in accordance with any one of the preceding claims, **characterized in that** the field changing member (68) is at least partially magnetically polarizable and has a magnetic susceptibility χₘ differing from zero.

6. Push-button unit in accordance with claim 5, **characterized in that** the field changing member (68) is at least partially diamagnetic, paramagnetic, ferromagnetic, antiferromagnetic or ferrimagnetic.

7. Push-button unit in accordance with claim 5 or 6, **characterized in that** the field changing member is a soft iron element (68).

8. Push-button unit in accordance with any one of the preceding claims, **characterized in that** the field generating unit is a magnet (62).

9. Push-button unit in accordance with claim 8, **characterized in that** the magnet is a permanent magnet (62).

10. Push-button unit in accordance with claim 8, **characterized in that** the magnet (62) is formed by a coil.

11. Push-button unit in accordance with any one of the preceding claims, **characterized in that** the actuating sensor (50) is disposed between poles of the field generating unit (62).

12. Push-button unit in accordance with claim 11, **characterized in that** the actuating sensor (50) is disposed in a gap of a ring coil.

13. Push-button unit in accordance with any one of the preceding claims, **characterized in that** a cross section (78, 80) of the field changing member (68) varies in a direction of actuation (70) of the actuating member (22, 24).

14. Push-button unit in accordance with claim 13, **characterized in that** the cross section (78, 80) increases.

15. Push-button unit in accordance with any one of the preceding claims, **characterized in that** the return path system (56) is a magnetic return path system.

16. Push-button unit in accordance with any one of the preceding claims, **characterized in that** the field generating unit (62), the return path system (56) and the actuating sensor (50) define a recess (64), and **in that** the field changing member (68) is so disposed that it is at least partially introducible into the recess (64) as a result of a movement of the actuating member (22, 24).

17. Push-button unit in accordance with claim 16, **characterized in that** the recess (64) has a substantially rectangular cross section.

18. Push-button unit in accordance with any one of the preceding claims, **characterized in that** the actuating member (22, 24) carries the field changing member (68).

19. Push-button unit in accordance with any one of the preceding claims, **characterized in that** the drive unit (10) has at least two different operating modes, a first operating mode position of the at least one actuating member being associated with a first operating mode, and **in that** the at least one actuating member (22, 24) is rotatable about an axis of rotation (70) from the first operating mode position to a second operating mode position, which is associated with a second operating mode of the drive unit (10), in order to switch the drive unit (10) over from the first operating mode to the second operating mode.

20. Push-button unit in accordance with claim 19, **characterized in that** two actuating sensors (50) are provided and disposed so that there is detectable with the one actuating sensor (50) a position of the at least one actuating member (22, 24) in the first operating mode position or a movement of the at least one actuating member (22, 24) in the first operating mode position, and with the second actuating sensor a position of the at least one actuating member (22, 24) in the second operating mode position or a movement of the at least one actuating member (22, 24) in the second operating mode position.

21. Surgical drive unit (10) comprising a surgical drive (14) and a push-button unit (20) for prescribing a rotational speed and/or a direction of rotation of the drive unit, **characterized in that** the push-button unit (20) is a push-button unit (20) in accordance with any one of the preceding claims.

22. Drive unit in accordance with claim 21, **characterized in that** the drive unit (10) is operated by a battery or an accumulator.

23. Drive unit in accordance with claim 21 or 22, **characterized in that** a control and/or regulating unit is provided for controlling and/or regulating a rotational speed and/or an operating mode of the drive unit (10).

24. Drive unit in accordance with claim 23, **characterized in that** the drive unit (10) comprises the control and/or regulating unit.

## Revendications

1. Unité chirurgicale à poussoirs de commande (20) pour prescrire une vitesse de rotation et/ou un sens de rotation d'une unité d'entraînement chirurgicale (10), comprenant au moins un organe d'actionnement (22, 24) monté mobile dans une direction d'actionnement (70), une unité de production de champ (62) pour produire un champ magnétique ou électrique, et au moins un détecteur d'actionnement (50) pour produire un signal d'actionnement en réaction à un mouvement et/ou à une position de l'organe d'actionnement (22, 24), le signal d'actionnement produit étant en corrélation avec une intensité de champ et/ou une variation du champ produit par l'unité de production de champ (62) suite à un mouvement de l'organe d'actionnement (22, 24), **caractérisée en ce que** le détecteur d'actionnement (50) est couplé à l'unité de production de champ (62), **en ce qu'**il est prévu un organe de variation de champ (68) pour engendrer une variation du champ agissant à l'endroit du détecteur d'actionnement (50) et produit par l'unité de production de champ (62), suite à un mouvement et/ou à une position modifiée de l'organe d'actionnement (22, 24), et **en ce que** l'unité de production de champ (62) est couplée au détecteur d'actionnement (50) par l'intermédiaire d'un système de bouclage de retour (56).

2. Unité à poussoirs de commande selon la revendication 1, **caractérisée en ce que** le détecteur d'actionnement (50) et l'unité de production de champ (62) sont agencés de manière fixe l'un par rapport à l'autre.

3. Unité à poussoirs de commande selon l'une des revendications précédentes, **caractérisée en ce que** le détecteur d'actionnement est un détecteur à champ magnétique (50).

4. Unité à poussoirs de commande selon la revendication 3, **caractérisée en ce que** le détecteur à champ magnétique est un détecteur de Hall (50).

5. Unité à poussoirs de commande selon l'une des revendications précédentes, **caractérisée en ce que** l'organe de variation de champ (68) peut être au moins partiellement polarisé magnétiquement, et présente une susceptibilité magnétique χₘ différente de zéro.

6. Unité à poussoirs de commande selon la revendication 5, **caractérisée en ce que** l'organe de variation de champ (68) est au moins partiellement diamagnétique, paramagnétique, ferromagnétique, antiferromagnétique ou ferrimagnétique.

7. Unité à poussoirs de commande selon la revendication 5 ou 6, **caractérisée en ce que** l'organe de variation de champ (68) est un élément de fer doux (68).

8. Unité à poussoirs de commande selon l'une des revendications précédentes, **caractérisée en ce que** l'unité de production de champ est un aimant (62).

9. Unité à poussoirs de commande selon la revendication 8, **caractérisée en ce que** l'aimant est un aimant permanent (62).

10. Unité à poussoirs de commande selon la revendication 8, **caractérisée en ce que** l'aimant (62) est formé par une bobine.

11. Unité à poussoirs de commande selon l'une des revendications précédentes, **caractérisée en ce que** le détecteur d'actionnement (50) est agencé entre des pôles de l'unité de production de champ (62).

12. Unité à poussoirs de commande selon la revendication 11, **caractérisée en ce que** le détecteur d'actionnement (50) est agencé dans un interstice ou entrefer d'une bobine annulaire.

13. Unité à poussoirs de commande selon l'une des revendications précédentes, **caractérisée en ce qu'**une section transversale (78, 80) de l'organe de variation de champ (68) varie dans une direction d'actionnement (70) de l'organe d'actionnement (22, 24).

14. Unité à poussoirs de commande selon la revendication 13, **caractérisée en ce que** la section transversale (78, 80) augmente.

15. Unité à poussoirs de commande selon l'une des revendications précédentes, **caractérisée en ce que** le système de bouclage de retour (56) est un système de bouclage de retour magnétique.

16. Unité à poussoirs de commande selon l'une des revendications précédentes, **caractérisée en ce que** l'unité de production de champ (62), le système de bouclage de retour (56) et le détecteur d'actionnement (50) définissent un évidement (64), et **en ce que** l'organe de variation de champ (68) est agencé de manière à pouvoir être introduit au moins partiellement dans l'évidement (64), suite à un mouvement de l'organe d'actionnement (22, 24).

17. Unité à poussoirs de commande selon la revendication 16, **caractérisée en ce que** l'évidement (64) présente une section transversale sensiblement de forme rectangulaire.

18. Unité à poussoirs de commande selon l'une des revendications précédentes, **caractérisée en ce que** l'organe d'actionnement (22, 24) porte l'organe de variation de champ (68).

19. Unité à poussoirs de commande selon l'une des revendications précédentes, **caractérisée en ce que** l'unité d'entraînement (10) présente au moins deux modes de fonctionnement différents, une première des positions de modes de fonctionnement dudit au moins un organe d'actionnement étant associée à un premier mode de fonctionnement, et **en ce que** ledit au moins un organe d'actionnement (22, 24) peut être tourné autour d'un axe de rotation (70) de la première des positions de modes de fonctionnement dans la deuxième des positions de modes de fonctionnement, qui est associée à un deuxième mode de fonctionnement de l'unité d'entraînement (10), en vue de commuter l'unité d'entraînement (10) du premier mode de fonctionnement dans le deuxième mode de fonctionnement.

20. Unité à poussoirs de commande selon la revendication 19, **caractérisée en ce que** sont prévus deux détecteurs d'actionnement (50) agencés de façon à pouvoir détecter avec un détecteur d'actionnement (50) une position dudit au moins un organe d'actionnement (22, 24) dans la première des positions de modes de fonctionnement ou bien un mouvement dudit au moins un organe d'actionnement (22, 24) dans la première des positions de modes de fonctionnement, et de façon à pouvoir détecter avec le deuxième détecteur d'actionnement, une position dudit au moins un organe d'actionnement (22, 24) dans la deuxième des positions de modes de fonctionnement ou bien un mouvement dudit au moins un organe d'actionnement (22, 24) dans la deuxième des positions de modes de fonctionnement.

21. Unité d'entraînement chirurgicale (10) comprenant un entraînement chirurgical (14) et une unité à poussoirs de commande (20) pour prescrire une vitesse de rotation et/ou un sens de rotation de l'unité d'entraînement, **caractérisée en ce que** l'unité à poussoirs de commande (20) est une unité à poussoirs de commande (20) selon l'une des revendications précédentes.

22. Unité d'entraînement selon la revendication 21, **caractérisée en ce que** l'unité d'entraînement (10) fonctionne sur piles ou batteries d'accumulateurs.

23. Unité d'entraînement selon l'une des revendications 21 ou 22, **caractérisée en ce qu'**il est prévu une unité de commande et/ou de régulation pour commander et/ou réguler une vitesse de rotation et/ou un mode de fonctionnement de l'unité d'entraînement (10).

24. Unité d'entraînement selon la revendication 23, **caractérisée en ce que** l'unité d'entraînement (10) englobe l'unité de commande et/ou de régulation.
